(19) Europäisches Patentamt
European Patent Office
Office européen des brevets

(11) Numéro de publication : 0 307 303 B1

# (12) FASCICULE DE BREVET EUROPEEN

(45) Date de publication du fascicule du brevet : 04.12.91 Bulletin 91/49

(51) Int. Cl.[5] : C07D 401/12, C07D 409/14, C07D 409/04, C07D 211/70, A61K 31/505

(21) Numéro de dépôt : 88402242.7

(22) Date de dépôt : 07.09.88

## (54) [(Pyrimidinyl-2)-aminoalkyl]-1 pipéridines, leur préparation et leur application en thérapeutique.

Le dossier contient des informations techniques présentées postérieurement au dépôt de la demande et ne figurant pas dans le présent fascicule.

(30) Priorité : 09.09.87 FR 8712502

(43) Date de publication de la demande : 15.03.89 Bulletin 89/11

(45) Mention de la délivrance du brevet : 04.12.91 Bulletin 91/49

(84) Etats contractants désignés : AT BE CH DE ES FR GB GR IT LI LU NL SE

(56) Documents cités : EP-A- 0 037 713

(73) Titulaire : SYNTHELABO
58 rue de la Glacière
F-75013 Paris (FR)

(72) Inventeur : Manoury, Philippe
L'Orée de Verrières 38, Avenue des Vaupépins
F-91370 Verrières le Buisson (FR)
Inventeur : Saarmets, Alfred
35Bis, Rue du Moulin à Vent
F-94370 Sucy en Brie (FR)

(74) Mandataire : Thouret-Lemaitre, Elisabeth et al
SYNTHELABO Service Brevets 22, avenue Galilée
F-92352 LE PLESSIS ROBINSON CEDEX (FR)

EP 0 307 303 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés pyrimidinylaminés de pipéridine, leur préparation et leur application en thérapeutique.

Des composés analogues à ceux de la présente invention, le cyproheptadin et le pizotifen, sont déjà connus comme antagonistes 5-HT2.

Les composés de l'invention répondent à la formule (I) donnée dans l'annexe 1, dans laquelle

X représente un radical $(CH_2)2$, CH = CH ou $CH_2$-CO,

Y représente le radical CH = CH ou un atome de soufre,

n est 2,3 ou 4,

$R_1$ est un atome d'hydrogène ou d'halogène,

$R_2$ est un atome d'hydrogène ou un radical $(C_{1-4})$alkyle,

$R_3$ est un atome d'hydrogène ou le radical hydroxy.

Les sels que forment les composés (I) avec les acides pharmaceutiquement acceptables font partie de l'invention.

Selon l'invention, les composés (I) peuvent être préparés selon le schéma réactionnel donné dans l'annexe 1 :

on fait réagir un composé de formule (II) avec un composé de formule $R(CH_2)_nZ$ dans laquelle Z est un groupe labile et R est un groupe phtalimido ou N(Alk)(Trityl), Alk étant un radical $(C_{1-4})$alkyle, dans un solvant tel que la méthylisobutyl-cétone, à une température de 80 à 130°C, puis soit on fait réagir le composé (III) avec l'hydrazine pour obtenir le composé (V) soit on hydrolyse le composé (IV) en composé (VI) et enfin on fait réagir le composé (V) ou (VI) avec un composé de formule (VII) dans un solvant tel que la méthylisobutylcétone ou le toluène, à une température de 80 à 130°C, pour obtenir le composé (I).

Lorsque $R_2$ est un radical méthyle, une variante consiste à faire réagir le composé (V) avec l'anhydride trifluoroacétique pour le transformer en composé (VIII) qu'on fait réagir avec de l'iodure de méthyle pour obtenir le composé (VI).

Les composés de départ (II) sont soit décrits dans la littérature sont obtenus à partir de composés décrits dans la littérature selon les schémas réactionnels décrits en annexe 2.

Les composés $Z-(CH_2)_n-N(CH_3)$(Trityl) sont décrits par J.D. Billimoria et K.O. Lewis, J. Chem. Soc. Chem. Comm. 1404. (1968).

Les exemples suivants illustrent l'invention. Les spectres IR et RMN confirment la structure des composés.

Exemple 1. (Dihydro-10,11 fluoro-3 5H-dibenzo[a,d]cycloheptényIidène-5)-4 [[N-méthyl (N-pyrimidinyl-2)amino]-2 éthyl]-1 pipéridine.

1.1. Dihydro-10,11 fluoro-3 (méthyl-1 pipéridinyl-4)-5 5H dibenzo[a,d]cycloheptènol-5.

Dans un réacteur de 4 l contenant du magnésium finement broyé (40 g, 1,65 mol) on introduit du tétrahydrofuranne (THF), jusqu'à recouvrir légèrement le magnésium, un cristal d'iode puis, de la chloro-4 méthyl-1 pipéridine pure (2-3 ml). Lorsqu'une réaction vive se produit, on commence l'agitation et l'addition de la chloro-4 méthyl-1 pipéridine en solution dans du THF (210 g, 1,57 mol, 200 ml de THF), à un rythme tel que le reflux du THF soit maintenu. Lorsque l'addition est terminée, on ajoute 500 ml de THF et poursuit le reflux pendant 2 heures. Le mélange réactionnel est ensuite refroidi à 5°C. On ajoute alors, goutte à goutte, la solution de dihydro-10,11 fluoro-3 5H-dibenzo[a,d]cycloheptènone-5 (175 g, 0,775 mol, 750 ml de THF) (demande de brevet britannique 2132618) puis on laisse revenir lentement le mélange à la température ambiante et poursuit l'agitation à la température ambiante pendant 2 heures. Le réacteur est alors placé dans un mélange réfrigérant (−10°C), et le magnésien est hydrolysé, par addition lente d'une solution aqueuse saturée de chlorure d'ammonium (190 ml) ; on agite 1 heure à la température ambiante, filtre, lave avec du THF, et évapore le filtrat à sec. On reprend l'huile obtenue par du chlorure de méthylène, lave à l'eau, sèche, évapore, redissout l'huile dans de l'éther (500 ml) et ajoute goutte à goutte de l'éther chlorhydrique (500 ml ; 4 mol/l), agite 1 h à la température ambiante, filtre le produit précipité, le lave avec de l'éther puis de l'eau puis à nouveau avec de l'éther. Le chlorhydrate ainsi obtenu est ajouté à une solution aqueuse de soude (1000 ml, 2,5 mol/l). On extrait la base par du chlorure de méthylène, lave à l'eau, sèche, évapore et cristallise le produit dans de l'hexane. On fait recristalliser le produit dans de l'isopropanol. F = 175°C.

1.2. Dihydro-10,11 fluoro-3 (cyano-1 pipéridinyl-4)-5 5H-dibenzo[a,d]cycloheptènol-5.

A une solution de bromure de cyanogène (30 g, 0,28 mol) dans du benzène rigoureusement sec (1000

ml), on ajoute le produit obtenu à l'étape précédente (83 g, 0,26 mol) par petites portions, à l'aide d'un entonnoir à poudres ; le milieu réactionnel est ensuite agité 10 h à la température ambiante. On évapore le benzène et utilise le produit brut obtenu, (F = 250°C) tel quel dans l'étape suivante.

1.3. (Dihydro-10,11 fluoro-3 5H-dibenzo[a,d]cycloheptènylidène-5)-4 pipéridine.

Après avoir mis le cyanamide brut précédent (86 g, 0,256 mol) en suspension dans une solution aqueuse d'acide acétique (2 l d'acide acétique, 1,5 l d'eau), on ajoute goutte à goutte 260 ml d'acide chlorhydrique concentré puis on chauffe à la température du reflux pendant 10 h (passage en solution à 80°C). On concentre à demi-volume sous pression reduite, place au refrigérateur 2 heures, filtre, lave avec de l'eau, de l'éther et sèche. On obtient le produit sous forme de chlorhydrate blanc. F = 340°C. Base ; F = 105°C.

1.4. (Dihydro-10,11 fluoro-3 5H-dibenzo[a,d]cycloheptènylidène-5)-4 (N-méthylamino-2 éthyl)-1 pipéridine.

On chauffe à la température du reflux un mélange de (dihydro-10,11 fluoro-3 5H-dibenzo[a,d]cycloheptènylidène-5)-4 pipéridine (5 g, 0,017 mol) et d'iodure de [(N-méthyl N-triphénylméthyl)amino]-2 éthyle (7,3 g, 0,017 mol) dans 30 ml de méthylisobutylcétone (MiBC). On ajoute à cette température, en une fois, le carbonate de potassium (4,7 g, 0,034 mol), puis on chauffe le mélange à la température du reflux sous atmosphère d'argon, pendant 10 h. On refroidit, filtre et évapore à sec. On reprend ensuite l'huile résiduelle avec du chlorure de méthylène, lave à l'eau, à l'eau salée, sèche sur sulfate de magnésium puis évapore à sec. On ajoute à l'huile obtenue 100 ml d'acide chlorhydrique 1N, puis agite 5 heures à la température ambiante. On sépare le tritylméthanol par filtration, neutralise les eaux mères avec de la soude aqueuse 2N, extrait le produit à l'aide de chlorure de méthylène, sèche sur sulfate de magnésium et évapore. On utilise l'huile obtenue sans purification supplémentaire dans l'étape suivante.

1.5. (Dihydro-10,11 fluoro-3 5H-dibenzo[a,d]cycloheptènylidène-5)-4 [[N-méthyl N-(pyrimidinyl-2)amino]-2 éthyl-1 pipéridine.

On chauffe à la température du reflux un mélange du composé obtenu à l'étape précédente (4,7 g, 0,0135 mol) et de chloro-2 pyrimidine (1,7 g 0,0148 mol) dans 25 ml de MiBC. On ajoute à cette température, en une seule fois, l'hydrogénocarbonate de sodium (2,5 g, 0,03 mol) et on chauffe le mélange à la température du reflux durant 8 heures, sous atmosphère d'argon. On filtre, lave l'insoluble minéral avec du chlorure de méthylène et évapore à sec. On chromatographie l'huile résiduelle sur silice 60 Merck en éluant par un mélange chlorure de méthylène/acétone (50/50, v/v). On évapore les solvants des fractions qui contiennent le produit. On ajoute la quantité stoechiométrique d'acide maléïque en solution alcoolique, à la solution de la base brute dans de l'acétate d'éthyle. On filtre après une nuit de repos le maléate du produit cherché.

Le produit cristallisé contient 1,5 mole d'acide maléique par mole de base. F = 135°C.

Exemple 2.(Fluoro-3 5H-dibenzo[a,d]cycloheptènylidène-5)-4 [[N-méthyl N-(pyrimidinyl-2)amino]-2 éthyl]-1 pipéridine.

2.1. Fluoro-3(méthyl-1 pipéridinyl-4)-5 5H-dibenzo[a,d]cyclohepténol-5.

On prépare ce composé à partir de la fluoro-3 5H-dibenzo [a,d]cyclohepténone-5 (demande de brevet britannique 2132618) selon la méthode décrite dans l'exemple 1.1.
F = 300°C (chlorhydrate).

2.2. Fluoro-3 (cyano-1 pipéridinyl-4)-5 5H-dibenzo[a,d]cyclohepténol-5.

A partir du composé précédent 2.1., en utilisant la méthode de synthèse de l'exemple 1.2., on obtient ce composé.
F = 280°C.

2.3. (Fluoro-3 5H-dibenzo[a,d]cycloheptènylidène-5)-4 pipéridine.

On hydrolyse le cyanamide précédent 2.2. dans les conditions décrites dans l'exemple 1.3.
F > 300°C (chlorhydrate) F = 161°C (base).

2.4. (Fluoro-3 5H-dibenzo[a,d]cycloheptènylidène-5)-4 [(N-méthyl-amino)-2 éthyl]-1 pipéridine.

On prépare ce composé à partir de la fluoro-3 5H-dibenzo[a,d]cycloheptènylidène-5)-4 pipéridine (2,5 g, 0,0085 mol) selon le mode opératoire utilisé pour la synthèse du composé décrit dans l'exemple 1.4. On l'utilise tel quel dans l'étape suivante.

2.5. (Fluoro-3 5H-dibenzo[a,d]cycloheptènylidène-5)-4 [(N-méthylN-(pyrimidinyl-2)amino]-2 éthyl]-1 pipéridine.

A partir du composé précédent 2.4. (2 g, 0,0057 mol) et de chloro-2 pyrimidine (0,7 g, 0,061 mol), selon la méthode d'alkylation décrite pour le dérivé de l'exemple 1.5, on obtient le composé cherché sous forme de base. On prépare le maléate. F = 184°C.

Exemple 3. (5H-dibenzo[a,d]cycloheptényIidène-5)-4 [[N-(pyrimidinyl-2)amino]-2 éthyl]-1 pipéridine.

3.1. (5H-dibenzo[a,d]cycloheptènylidène-5)-4 [(dioxo-1,3 isoindolinyl-2)-2 éthyl]-1 pipéridine.

On porte à la température du reflux un mélange de norcyproheptadine (Engelhardt et al, J. Med. Chem. 8 829 (1965) (10 g, 0,036 mol) et de (bromo-2 éthyl)-2 dioxy-1,3 isoindole (9,9 g, 0,039 mol) dans de la méthylisobutylcétone (200 ml). Après 10 mn d'agitation à la température du reflux on introduit le carbonate de potassium (11 g, 0,08 mol) en une fois. On poursuit le reflux pendant 8 h, filtre, évapore et purifie le produit brut obtenu par recristallisation dans du méthanol. On isole de cette façon le produit attendu, sous forme de base. F = 190°C.

3.2. (5H-dibenzo[a,d]cycloheptènylidène-5)-4 (amino-2 éthyl-1 pipéridine.

A une suspension du composé précédent 3.1. (26 g, 0,059 mol) dans du méthanol (250 ml), on ajoute, goutte à goutte, une solution méthanolique d'hydrazine (250 ml, 1,4 mol/l) puis on agite le mélange à la température ambiante pendant 8 heures. On évapore à sec (coévaporation avec trois fois 250 ml de méthanol) puis reprend le mélange ainsi obtenu avec du chlorure de méthylène, filtre,lave les eaux mères avec de la soude aqueuse 2N, de l'eau salée, sèche, évapore et fait cristalliser le produit dans du pentane.
F = 102°C (base).

3.3. (5H-dibenzo[a,d]cycloheptènylidène-5)-4 [[N-(pyrimidinyl-2)amino]-2 éthyl]-1 pipéridine.

On porte à la température du reflux, dans de la méthylisobutylcétone (10 ml), un mélange du composé 3.2. (1,3 g, 0,004 mol) et de chloro-2 pyrimidine (0,5 g, 0,004 mol), puis 10 mn après on ajoute du bicarbonate de sodium (0,4 g ; 0,005 mol). Le reflux est maintenu pendant 20 heures. On filtre, évapore le solvant et chromatographie l'huile résiduelle sur silice ($SiO_2$40 Merck, éluant : chlorure de méthylène/méthanol 9/1 v/v). Après évaporation on obtient la base brute dont on prépare le maléate que l'on fait recristalliser dans de l'acétate d'éthyle.
F = 198°C.

Exemple 4. (5H-dibenzo[a,d]cycloheptènylidène-5)-4 [[N-méthyl N-(pyrimidinyl-2)amino]-2 éthyl]-1 pipéridine et son maléate.

4.1. (5H-dibenzo[a,d]cycloheptènylidène-5)-4 [(N-méthyl-amino)-2 éthyl]-1 pipéridine.

On porte à la température du reflux, un mélange de norcyproheptadine (6,8 g ; 0,025 mol) et d'iodure de [(N-méthyl N-triphénylméthyl)amino]-2 éthyle (10,16 g, 0,025 mol) dans 50 ml de MiBC. On ajoute alors du carbonate de potassium (6,8 g, 0,050 mol) en une fois et chauffe le mélange à la température du reflux pendant 10 heures, sous atmosphère d'argon. Après avoir laissé refroidir, on filtre et évapore le filtrat à sec. On dissout l'huile obtenue dans du méthanol contenant de l'acide oxalique (3 g, 0,033 mol). On évapore à sec, reprend par de l'acide acétique aqueux (100 ml, 90 %), porte à reflux 30 mn, concentre à demi-volume et laisse au réfrigérateur. On filtre l'oxalate cristallisé et le sèche.
F = 205°C (décomposition).

4.2. (5H-dibenzo[a,d]cycloheptènylidène-5)-4 [[N-méthyl N-(pyrimidinyl-2)amino]-2 éthyl]-1 pipéridine et son maléate.

On porte à la température du reflux, un mélange du composé obtenu à l'étape précédente 4.1. (2,1 g, 0,006 mol) et de chloro-2 pyrimidine (0,8 g, 0,007 mol) dans 10 ml de MiBC. Après 10 mn de reflux on ajoute du bicarbonate de sodium (1 g, 0,0012 mol) et poursuit le reflux pendant 4 h. On filtre, évapore à sec, reprend par du chlorure de méthylène, lave à l'eau, l'eau salée, sèche sur sulfate de magnésium et évapo- re. On dissout l'huile obtenue dans de l'isopropanol, puis ajoute 0,7 g d'acide maléique en solution dans 10 ml de méthanol. On concentre aux 2/3 et laisse au repos 8 h. On filtre le produit cristallisé, le lave avec de l'éther et le sèche à 40°C sous pression réduite. On obtient le maléate.
F = 179°C.

Exemple 5. (5H-dibenzo[a,d]cycloheptènylidène-5)-4 [[N-méthyl-N-(pyrimidinyl-2)amino]-3 propyl]-1 pipéridine.

5.1. (5H-dibenzo[a,d]cycloheptènylidène-5)-4 [(dioxo-1,3 isoindolinyl-2)-3 propyl]-1 pipéridine.

A partir de norcyproheptadine et de (bromo-3 propyl)-2 dioxo-1,3 isoindole et en mettant en oeuvre la méthode de synthèse décrite dans l'exemple 3.1. on prépare le composé.
F = 266°C (chlorhydrate).

5.2. (5H-dibenzo[a,d]cycloheptènylidène-5)-4 (amino-3 propyl)-1 pipéridine.

On prépare ce produit à partir du composé précédent 5.1. selon le mode opératoire utilisé pour la synthèse du dérivé décrit dans l'exemple 3.2.
F = 95°C (base).

5.3. (5H-dibenzo[a,d]cycloheptènylidène-5)-4 [(N-trifluoroacétyl-amino)-3 propyl]-1 pipéridine.

A une solution du composé 5.2. (10 g, 0,03 mol) dans 50 ml de chloroforme, contenant 0,1 ml d'acide sulfurique concentré et refroidie à 0°C, on ajoute, goutte à goutte, de l'anhydride trifluoroacétique (4,5 ml, 0,032 mol) à l'aide d'une seringue. Lorsque l'addition est terminée, on agite 10 mn à 0°C puis ajoute 250 ml d'eau puis du bicarbonate de sodium par petites portions (20 g, 0,24 mol), décante la phase organique, lave cette dernière à l'eau salée puis la sèche. Après évaporation à sec, on fait cristalliser l'huile obtenue dans de l'éther n-butylique. F = 106°C.

5.4. (5H-dibenzo[a,d]cycloheptènylidène-5)-4 [(N-méthylamino)-3 propyl]-1 pipéridine.

On ajoute de l'éther chlorhydrique au produit obtenu précédemment dans l'exemple 5.3 (11 g, 0,026 mol) en solution dans 200 ml de méthanol, puis évapore à sec. Au chlorhydrate obtenu, on ajoute de l'acétone, chauffe à la température du reflux, puis ajoute, goutte à goutte, une solution d'iodure de méthyle dans de l'acétone (6 g, 0,042 mol dans 50 ml). Après 20 mn de reflux on ajoute, avec précaution, de la potasse pulvérisée (3,5 g, 0,062 mol) chauffe encore 30 mn à la température du reflux puis évapore à sec. On reprend le résidu dans 200 ml d'eau puis on chauffe à la température du reflux 30 mn, laisse refroidir et extrait par du chlorure de méthylène. On évapore le chlorure de méthylène et chromatographie sur silice ($SiO_2$40 Merck, éluant : mélange méthanol/ammoniaque concentrée 90/10, v/v). Après évaporation des solvants, on reprend l'huile avec du chlorure de méthylène, lave à l'eau, sèche et évapore. On obtient le produit cherché que l'on utilise tel quel dans l'étape suivante.

5.5. (5H-dibenzo[a,d]cycloheptènylidène-5)-4 [[N-méthyl-N-(pyrimidinyl-2)amino]-3 propyl]-1 pipéridine.

On prépare ce composé à partir du composé 5.4. et de chloro-2 pyrimidine selon la méthode de synthèse décrite dans l'exemple 4.2.
F = 129°C (base).

Tableau

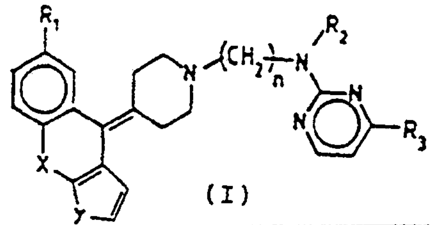

(I)

| Composé | n | $R_1$ | X | Y | $R_2$ | $R_3$ | Sel | F (°C) |
|---|---|---|---|---|---|---|---|---|
| 1 | 2 | H | CH=CH | CH=CH | H | H | maléate | 198 |
| 2 | 2 | H | CH=CH | CH=CH | $CH_3$ | H | maléate | 179 |
| 3 | 2 | H | CH=CH | CH=CH | H | OH | dichlorhydrate | 252 |
| 4 | 2 | H | CH=CH | CH=CH | $CH_3$ | OH | chlorhydrate | 200 |
| 5 | 2 | H | $CH_2-CH_2$ | CH=CH | $CH_3$ | H | maléate | 134 |
| 6 | 2 | F | CH=CH | CH=CH | $CH_3$ | H | maléate | 184 |
| 7 | 2 | F | $CH_2-CH_2$ | CH=CH | H | H | maléate | 149 |
| 8 | 2 | F | $CH_2-CH_2$ | CH=CH | $CH_3$ | H | maléate | 143 |
| 9 | 2 | F | $CH_2-CH_2$ | CH=CH | $CH_3$ | OH | maléate | 136 |
| 10 | 2 | Cl | $CH_2-CH_2$ | CH=CH | H | H | maléate | 154 |
| 11 | 2 | Cl | $CH_2-CH_2$ | CH=CH | $CH_3$ | H | maléate | 165 |
| 12 | 2 | Cl | $CH_2-CH_2$ | CH=CH | $CH_3$ | OH | maléate | 138 |
| 13 | 3 | H | CH=CH | CH=CH | H | H | base | 95 |
| 14 | 3 | H | CH=CH | CH=CH | $CH_3$ | H | base | 129 |
| 15 | 2 | H | $CH_2-CO$ | S | $CH_3$ | H | oxalate | 192 |
| 16 | 2 | H | $CH_2-CH_2$ | S | $CH_3$ | H | oxalate | 205 |

Tableau (suite)

| Composé | n | $R_1$ | X | Y | $R_2$ | $R_3$ | Sel | F (°C) |
|---|---|---|---|---|---|---|---|---|
| 17 | 2 | H | $CH_2$-CO | S | $CH_3$ | OH | oxalate | 140(dec) |
| 18 | 3 | Cl | $CH_2$-$CH_2$ | CH=CH | $CH_3$ | H | maléate | 147 |
| 19 | 4 | Cl | $CH_2$-$CH_2$ | CH=CH | $CH_3$ | H | maléate | 151 |

Les composés de l'invention possèdent une activité antisérotonine, (au niveau des récepteurs de type 5HT2).

Cette activité a été mise en évidence "in vitro" par déplacement de ligands fixés spécifiquement sur les récepteurs sérotoninergiques (test de binding SBS) et "in vivo" par antagonisme des effets de la sérotonine au niveau périphérique (test OES) et au niveau central (test AHT).

Test SBS : les composés de l'invention ont fait l'objet d'un essai de déplacement de la liaison (binding) du spiropéridol sur les récepteurs sérotoninergiques (5-HT2) du cortex cérébral du rat.

Pour cet essai on prélève les cerveaux de rats, on en dissèque le cortex et on l'homogénéise à 0°C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000 xg pendant 10 mn puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.

On soumet alors le tissu à une incubation préalable de 10 mn à 37°C en présence de 10 micromoles/l de pargyline, puis à une incubation de 20 mn à 37°C en présence de $^3$H-spiropéridol (activité spécifique : 25,6 Ci par millimole) à la concentration de 0,3 nanomole/l et de composé à étudier à des concentrations allant de 0,0001 à 100 micromoles/l.

On prélève des aliquots de 1 ml que l'on filtre sous vide, on lave les filtres deux fois avec 5 ml de tampon froid et on les sèche. La radioactivité est mesurée dans le toluène en présence de 5 g/l de diphényl-2,5 oxazole (PPO) et 0,1 g/l de bis-(phényl-5 oxazolyl-2)-1,4 benzène (POPOP).

Pour évaluer l'activité des composés on établit la courbe du pourcentage d'inhibition de la liaison spécifique de $^3$H-spiropéridol en fonction de la concentration en drogue déplaçante. On détermine graphiquement la concentration $IC_{50}$, concentration qui inhibe 50% de la liaison spécifique.

La liaison spécifique est définie comme étant la liaison déplacée par 100 micromoles/l de 5-HT.

Les concentrations $IC_{50}$ des composés de l'invention se situent pour la plupart entre 1 et 50 nanomoles/l.

Test OES : l'activité antisérotoninergique des composés de l'invention a également été mise en évidence par leur effet sur l'oedème provoqué chez le rat par la sérotonine, selon la méthode décrite par Maling et coll., J. Pharmacol. Exp. Therap., 191 (2), 300-310 (1974).

Les animaux sont des rats mâles de souche CD (Ch. River, France) pesant 120 à 150 g, à jeun depuis 18 h et répartis en blocs randomisés.

Les composés, mis en solution ou en suspension dans du Tween 80$^R$ à 1%, sont administrés par voie orale à raison de 0,5 ml pour 100 g de poids corporel, 1 h avant l'injection sous-plantaire, dans l'une des pattes postérieures, de 1 µg de sérotonine (en solution dans du sérum physiologique stérile, sous un volume de 0,1 ml). Le volume de l'oedème est mesuré 1 h après l'injection de sérotonine au moyen d'un pléthysmomètre à mercure Ugo Basile. La $DA_{40}$ (dose qui diminue de 40% le volume de l'oedème, par rapport aux animaux témoins) est déterminée graphiquement.

La $DA_{40}$ des composés de l'invention, déterminée par voie orale est comprise entre 0,1 et 2 mg/kg.

Test AHT : l'activité antisérotoninergique des composés a été étudiée quant à leur effet sur l'antagonisme des "head-twitches" (ébrouements de la tête) provoqués par le L-5-hydroxy-tryptophane (L-5-HTP) chez la souris, selon la méthode décrite par Corne et coll., Br. J. Pharmacol., 20, 106-120 (1962).

Les souris (mâles CD1, Charles River France ; 18-22 g de poids corporel) reçoivent les produits à étudier, à doses croissantes, ou le solvant, par voie intrapéritonéale ou orale, simultanément (voie i.p.) ou soixante

minutes avant (voie orale) une injection de L-5-HTP à la dose de 250 mg/kg par voie sous-cutanée. Quarante-cinq minutes après cette injection de 5-HTP, le nombre d'ébrouements est compté, pour chaque souris, pendant une minute.

Pour chaque traitement, la moyenne des ébrouements, ainsi que le pourcentage de variation par rapport au lot témoin, sont calculés.

A partir de la courbe effet-dose, on détermine la $DA_{50}$ (dose active 50% ou dose qui diminue de 50% le nombre moyen d'ébrouements par rapport aux animaux témoins) par la méthode graphique de Miller et Tainter (Proc. Soc. Exp. Biol. Med., (1944), 57, 261).

Les $DA_{50}$ des composés de l'invention se situent entre 0,05 et 2 mg/kg par voie intrapéritonéale et entre 0,1 et 4 mg/kg par voie orale.

Les composés de l'invention peuvent être utiles pour le traitement de la migraine, l'anxiété, la dépression, l'obésité, l'inflammation, l'asthme, les allergies, les spasmes vasculaires ou gastrointestinaux, l'hypertension et l'agrégation plaquettaire, et comme antiémétiques.

Certains composés possèdent également une activité antihistaminique.

Les composés de l'invention peuvent être administrés par voie orale ou parentérale, en association avec tout excipient approprié.

La posologie quotidienne peut aller de 5 à 200 mg.

Annexe 1

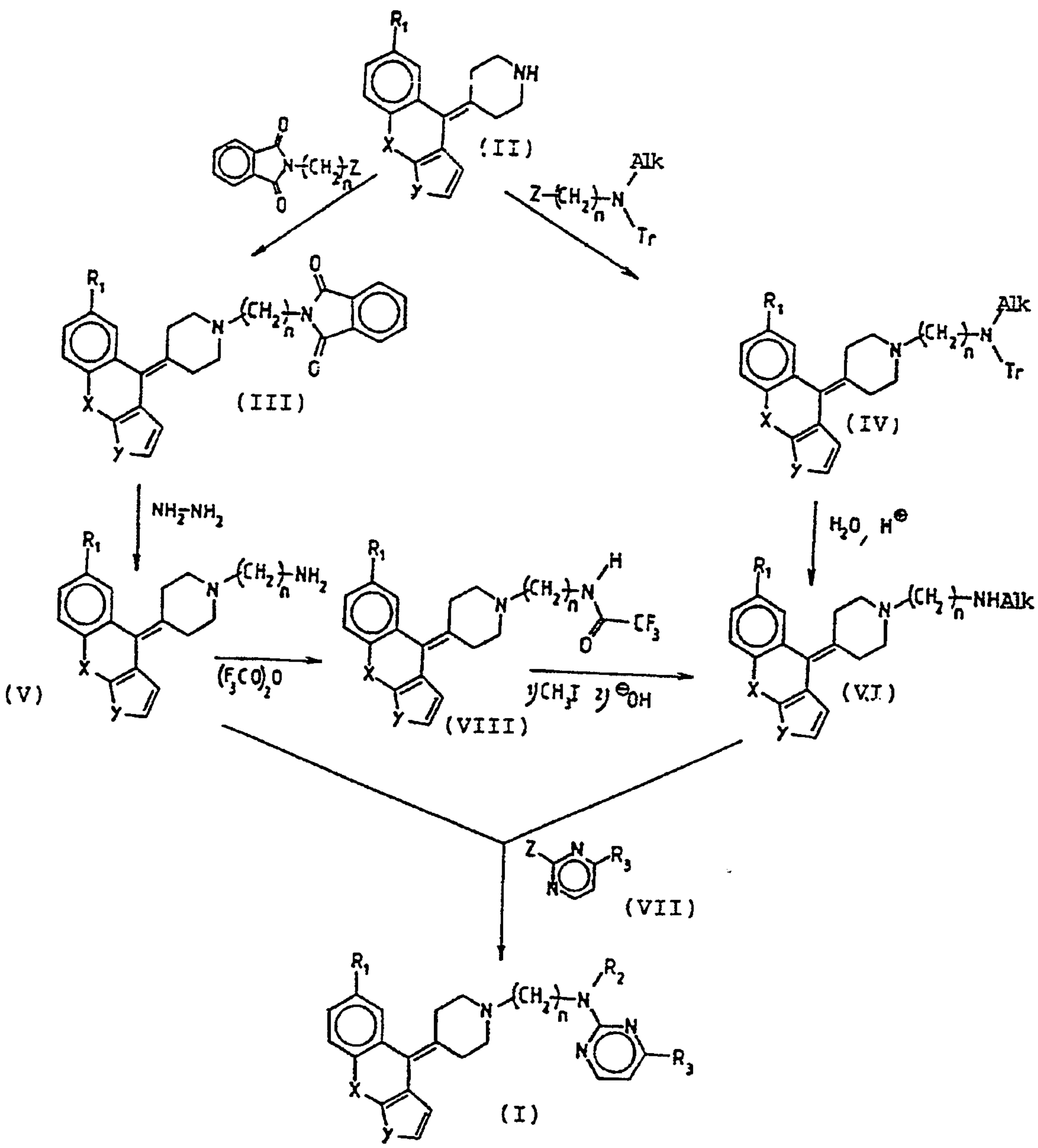

Annexe 2

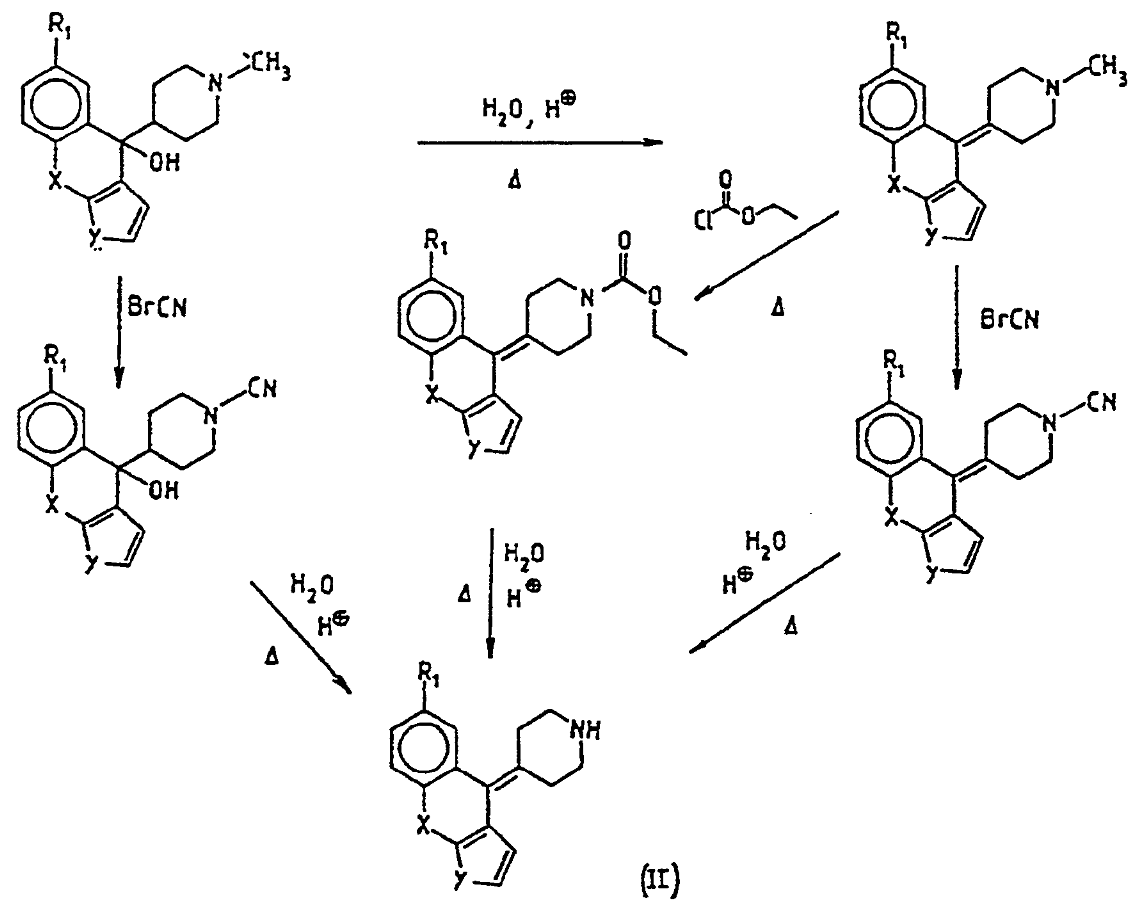


(II)

## Revendications

### Revendications pour les Etats suivants : AT, BE, CH, DE, FR, GB, IT, LI, LU, NL, SE

1. Dérivés pyrimidinylaminés de pipéridine, répondant à la formule (I)

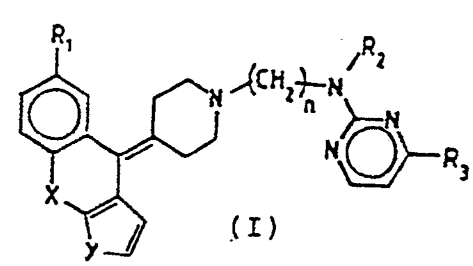

(I)

dans laquelle

X représente un radical $(CH_2)_2$, CH = CH ou $CH_2$-CO,

Y représente le radical CH = CH ou un atome de soufre,

n est 2, 3 ou 4,

$R_1$ est un atome d'hydrogène ou d'halogène,

$R_2$ est un atome d'hydrogène ou un radical $(C_{1-4})$alkyle,

$R_3$ est un atome d'hydrogène ou le radical hydroxy, ainsi que leurs sels d'addition aux acides pharmaceutiquement acceptables.

2. La dihydro-10,11 fluoro-3 5H- dibenzo[a,d]cycloheptényIidène-5)-4[[N-méthyl(N -pyrimidinyl-2)amino]-2 éthyl]-1 pipéridine, ainsi que ses sels d'addition aux acides pharmaceutiquement acceptables.

3. Procédé de préparation des composés selon la revendication 1, procédé caractérisé en ce que l'on fait réagir un composé de formule (II)

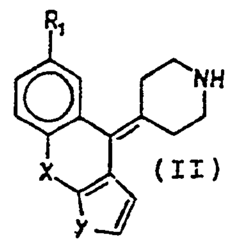

(II)

avec un composé de formule $R(CH_2)_nZ$ dans laquelle Z est un groupe labile et R est un groupe phtalimido ou N(Alk)(Trityl), Alk étant un radical $(C_{1-4})$alkyle, dans un solvant tel que la méthyl-isobutyl-cétone, à une température de 80 à 130°C, puis soit on fait réagir le composé (III)

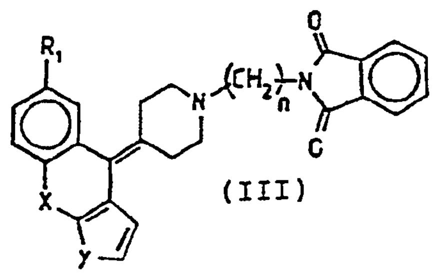

(III)

avec l'hydrazine pour obtenir le composé (V)

(V)

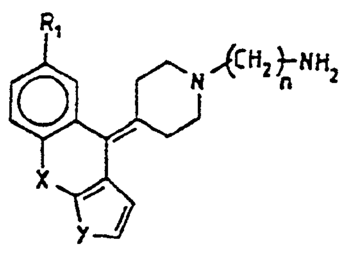

soit on hydrolyse le composé (IV)

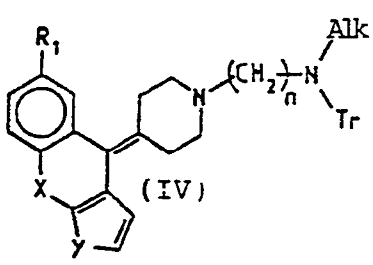

(IV)

en composé (VI)

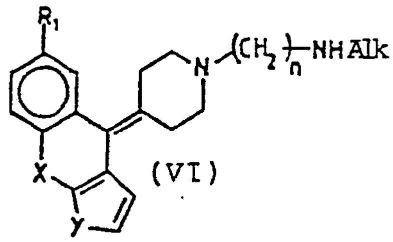
(VI)

et enfin on fait réagir le composé (V) ou (VI) avec un composé de formule (VII)

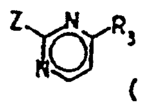
(VII)

dans un solvant tel que la méthylisobutylcétone, à une température de 80 à 130°C, pour obtenir le composé (I).

4. Médicament caractérisé en ce qu'il contient un composé tel que spécifié dans la revendication 1.

5. Composition pharmaceutique caractérisée en ce qu'elle contient un composé tel que spécifié dans la revendication 1 en association avec tout excipient approprié.

**Revendications pour les Etats suivants : ES, GR**

1. Procédé de préparation de dérivés pyrimidinylaminés de pipéridine, répondant à la formule (I)

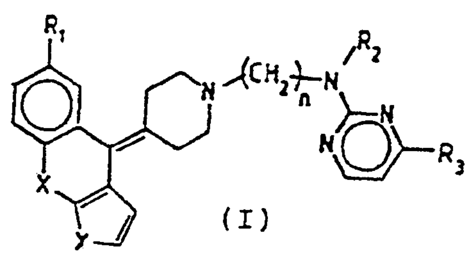
(I)

dans laquelle

X représente un radical $(CH_2)_2$, CH = CH ou $CH_2$-CO,

Y représente le radical CH = CH ou un atome de soufre,

n est 2, 3 ou 4,

$R_1$ est un atome d'hydrogène ou d'halogène,

$R_2$ est un atome d'hydrogène ou un radical $(C_{1-4})$alkyle,

$R_3$ est un atome d'hydrogène ou le radical hydroxy, ainsi que de leurs sels d'addition aux acides pharmaceutiquement acceptables, procédé caractérisé en ce que l'on fait réagir un composé de formule (II)

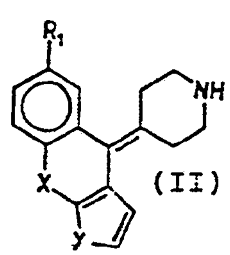
(II)

avec un composé de formule $R(CH_2)_nZ$ dans laquelle Z est un groupe labile et R est un groupe phtalimido ou N(Alk) (Trityl), Alk étant un radical $(C_{1-4})$alkyle, dans un solvant tel que la méthyl-isobutyl-cétone, à une température de 80 à 130°C, puis soit on fait réagir le composé (III)

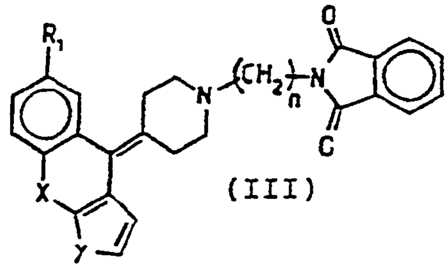

(III)

avec l'hydrazine pour obtenir le composé (V)

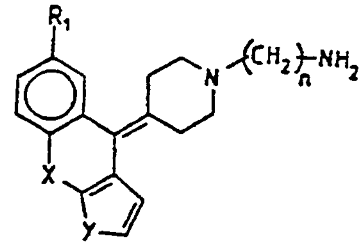

(V)

soit on hydrolyse le composé (IV)

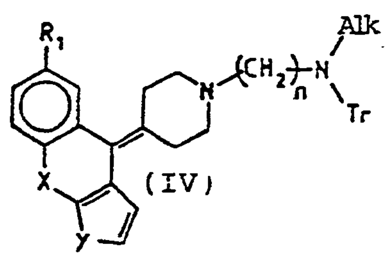

(IV)

en composé (VI)

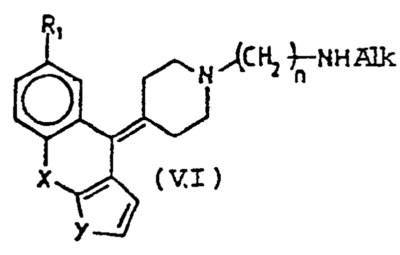

(VI)

et enfin on fait réagir le composé (V) ou (VI) avec un composé de formule (VII)

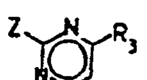

(VII)

dans un solvant tel que la méthylisobutylcétone, à une température de 80 à 130°C, pour obtenir le composé (I).

2. Procédé de préparation selon la revendication 1 du (dihydro-10, 11 fluoro-3 5H-dibenzo[a,d]cycloheptényIidène-5)-4[[N-méthyl(N-pyrimidinyl-2)amino]-2 éthyl]-1 pipéridine.

**Patentansprüche**

**Patentansprüche für folgende Vertragsstaaten : AT, BE, CH, DE, FR, GB, IT, LI, LU, NL, SE**

1. Pyrimidinylaminierte Piperidinderivate der Formel (I)

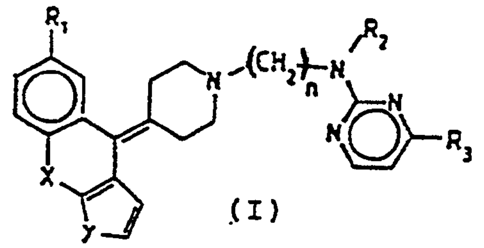

(I)

in welcher
X für einen $(CH_2)_2$—, CH=CH— oder $CH_2CO$— Rest,
Y für den CH=CH— Rest oder ein Schwefelatom,
n für die Zahl 2, 3 oder 4
$R_1$ für ein Wasserstoff- oder ein Halogenatom,
$R_2$ für ein Wasserstoffatom oder einen $(C_{1-4})$-Alkylrest,
$R_3$ für ein Wasserstoffatom oder den Hydroxyrest steht, sowie deren Additionssalze mit pharmazeutisch verwendbaren Säuren.

2. 4-(10,11-Dihydro-3-fluor-5H-dibenzo[a,d]cycloheptenyliden-5-)-1-[2-[N-methyl-(N-pyrimidinyl-2)-amino]-ethyl]-piperidin sowie dessen Additionssalze mit pharmazeutisch verwendbaren Säuren.

3. Verfahren zur Herstellung von Verbindungen nach Anspruch 1, **dadurch gekennzeichnet**, daß man eine Verbindung der Formel (II)

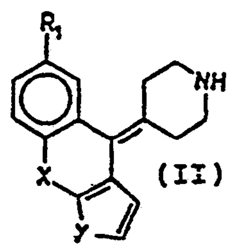

(II)

mit einer Verbindung der Formel $R(CH_2)_nZ$, in welcher Z für eine labile Gruppe steht und R eine Phthalimido- oder -N-(Alk) (Trityl)-Gruppe bedeutet, in welch letzterer Alk für einen $(C_{1-4})$-Alkylrest steht, in einem Lösungsmittel, wie in Methylisobutylketon, bei einer Temperatur von 80 bis 130°C reagieren läßt, dann entweder die Verbindung (III)

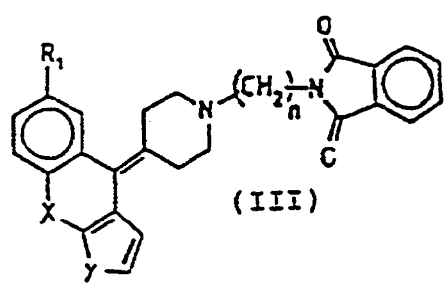

(III)

mit Hydrazin umsetzt, um die Verbindung (V)

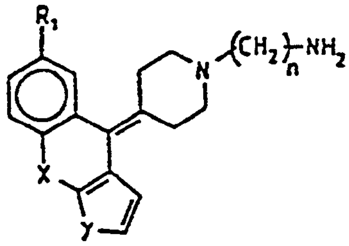

(V)

zu erhalten, oder die Verbindung (IV)

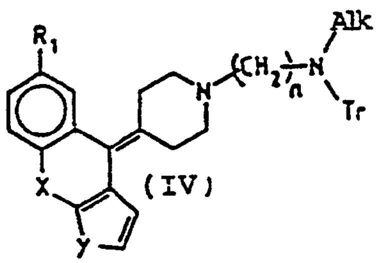

(IV)

zur Verbindung (VI)

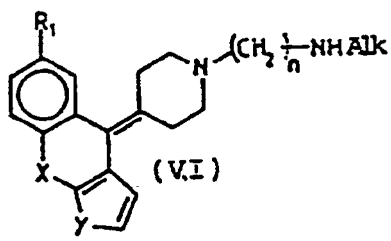

(V.I)

hydrolysiert und schließlich die Verbindung (V) oder (VI) mit einer Verbindung der Formel (VII)

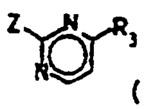

(VII)

in einem Lösungmittel, wie Methylisobutylketon, bei einer Temperatur von 80 bis 130°C reagieren läßt, um die Verbindung (I) zu gewinnen.

4. Arzneimittel, **dadurch gekennzeichnet**, daß es eine Verbindung nach Anspruch 1 enthält.

5. Pharmazeutische Zusammensetzung, **dadurch gekennzeichnet**, daß sie eine Verbindung nach Anspruch 1 in Kombination mit irgendeinem geeigneten Trägermittel enthält.

**Patentansprüche für folgende Vertragsstaaten : ES, GR**

1. Verfahren zur Herstellung von pyrimidinylaminierten Piperidinderivaten der Formel (I)

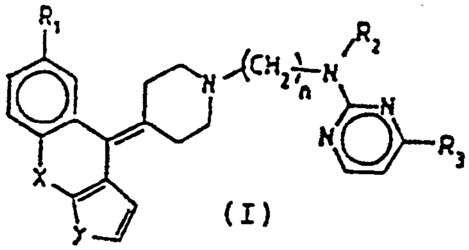

(I)

in welcher

X für einen $(CH_2)_2$—, CH=CH— oder $CH_2CO$— Rest,
Y für den CH=CH— Rest oder ein Schwefelatom,
n für die Zahl 2, 3 oder 4
$R_1$ für ein Wasserstoff- oder ein Halogenatom,
$R_2$ für ein Wasserstoffatom oder einen $(C_{1-4})$-Alkylrest,
$R_3$ für ein Wasserstoffatom oder den Hydroxyrest steht, sowie ihren Additionssalzen mit pharmazeutisch verwendbaren Säuren, **dadurch gekennzeichnet**, daß man eine Verbindung der Formel (II)

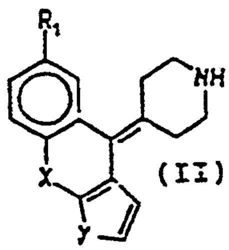

(II)

mit einer Verbindung der Formel $R(CH_2)_nZ$, in welcher Z für eine labile Gruppe steht und R eine Phthalimido- oder—N-(Alk) (Trityl)-Gruppe bedeutet, in welch letzterer Alk für einen $(C_{1-4})$-Alkylrest steht, in einem Lösungsmittel, wie in Methylisobutylketon, bei einer Temperatur von 80 bis 130°C reagieren läßt, dann entweder die Verbindung (III)

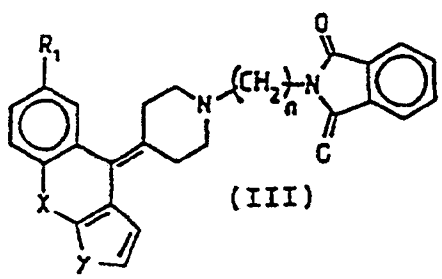

(III)

mit Hydrazin umsetzt, um die Verbindung (V)

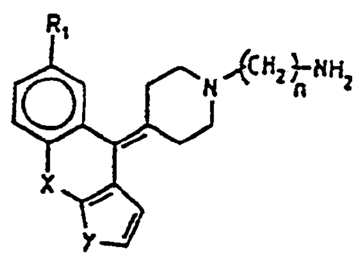

(V)

zu erhalten, oder die Verbindung (IV)

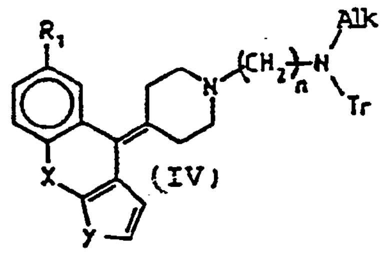

(IV)

zur Verbindung (VI)

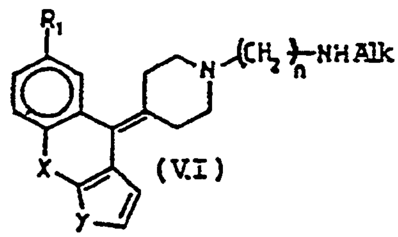

(VI)

hydrolysiert und schließlich die Verbindung (V) oder (VI) mit einer Verbindung der Formel (VII)

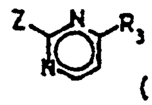

(VII)

in einem Lösungmittel, wie Methylisobutylketon, bei einer Temperatur von 80 bis 130°C reagieren läßt, um die Verbindung (I) zu gewinnen.

2. Verfahren nach Anspruch 1 zur Herstellung von 4-(10,11-Dihydro-3-fluor-5H-dibenzo[a,d]cycloheptenyliden-5-)-1-[2-[N-methyl-(N-pyrimidinyl-2)-amino]-ethyl]-piperidin.

## Claims

### Claims for the following Contracting States : AT, BE, CH, DE, FR, GB, IT, LI, LU, NL, SE

1. Pyrimidinylamino derivatives of piperidine, corresponding to the formula (I)

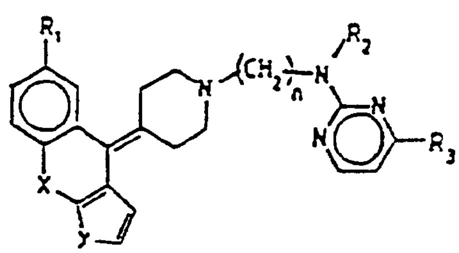

(I)

in which

X denotes a $(CH_2)_2$, CH=CH or $CH_2$-CO radical,
Y denotes a CH=CH radical or a sulphur atom,
n is 2, 3 or 4,
$R_1$ is a hydrogen or halogen atom,
$R_2$ is a hydrogen atom or a $(C_{1-4})$ alkyl radical, and
$R_3$ is a hydrogen atom or a hydroxy radical, as well as their addition salts with pharmaceutically acceptable acids.

2. 4-(10,11-Dihydro-3-fluoro-5H-dibenzo[a,d]cyclohepten-5-ylidene)-1-{2-[N-methyl-N-(2-Pyrimidinyl)amino]-ethyl}piperidine, as well as its addition salts with pharmaceutically acceptable acids.

3. Process for preparing the compounds according to Claim 1, which process is characterized in that a compound of formula (II)

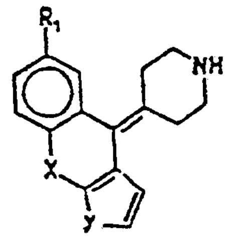

(II)

is reacted with a compound of formula $R(CH_2)_nZ$, in which Z is a labile group and R is a phthalimido or N(Alk)(Trityl) group, Alk being a $(C_{1-4})$ alkyl radical, in a solvent such as methyl isobutyl ketone, at a temperature of 80 to 130°C, then either the compound (III)

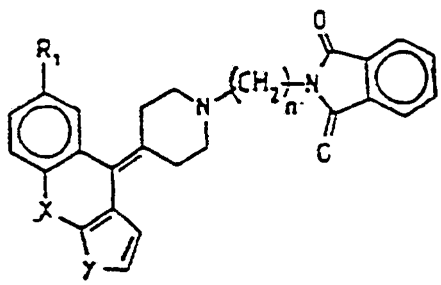

(III)

is reacted with hydrazine to obtain the compound (V)

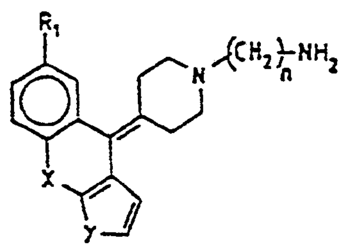

(V)

or the compound (IV)

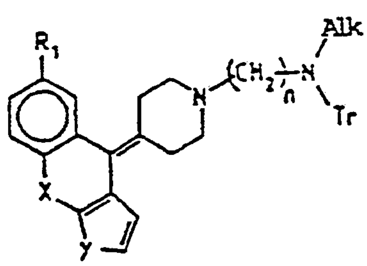

(IV)

is hydrolysed to the compound (VI)

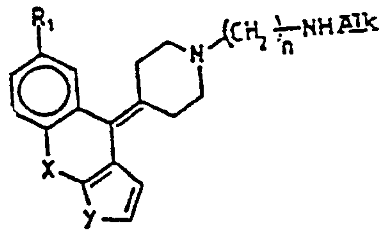

(VI)

and finally the compound (V) or (VI) is reacted with a compound of formula (VII)

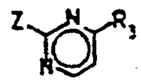

(VII)

in a solvent such as methyl isobutyl ketone, at a temperature of 80 to 130°C, to obtain the compound (I).

4. Medicinal product, characterized in that it contains a compound as specified in Claim 1.

5. Pharmaceutical composition, characterized in that it contains a compound as specified in Claim 1, in combination with any suitable excipient.

**Claims for the following Contracting States : ES, GR**

1. Process for preparing pyrimidinylamino derivatives of piperidine, corresponding to the formula (I)

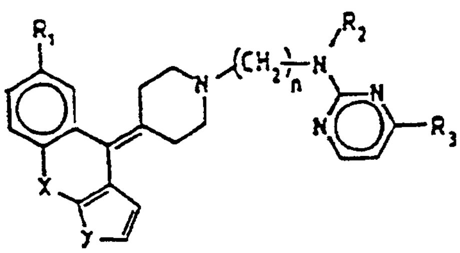

(I)

in which

X denotes a $(CH_2)_2$, CH=CH or $CH_2$-CO radical,
Y denotes a CH=CH radical or a sulphur atom,
n is 2, 3 or 4,
$R_1$ is a hydrogen or halogen atom,
$R_2$ is a hydrogen atom or a $(C_{1-4})$ alkyl radical, and
$R_3$ is a hydrogen atom or a hydroxy radical, as well as their addition salts with pharmaceutically acceptable acids, which process is characterized in that a compound of formula (II)

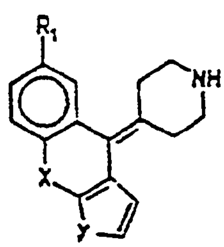

(II)

is reacted with a compound of formula $R(CH_2)_nZ$, in which Z is a labile group and R is a phthalimido or N(Alk)(Trityl) group, Alk being a $(C_{1-4})$ alkyl radical, in a solvent such as methyl isobutyl ketone, at a temperature of 80 to 130°C, then either the compound (III)

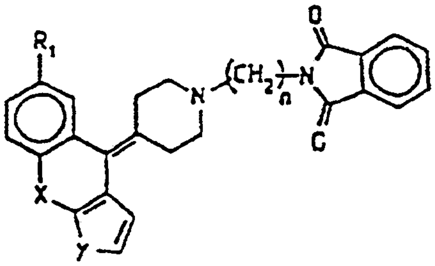

(III)

is reacted with hydrazine to obtain the compound (V)

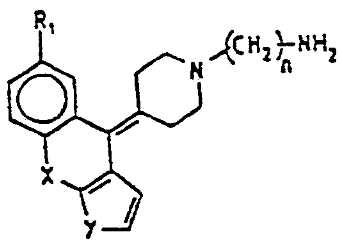

(V)

or the compound (IV)

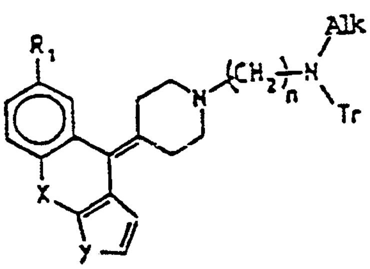

(IV)

is hydrolysed to the compound (VI)

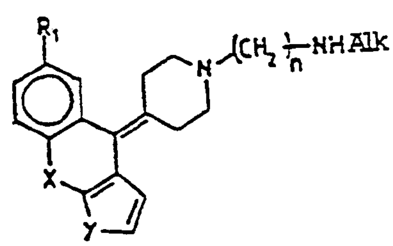

(VI)

and finally the compound (V) or (VI) is reacted with a compound of formula (VII)

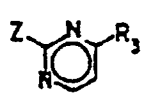

(VII)

in a solvent such as methyl isobutyl ketone, at a temperature of 80 to 130°C, to obtain the compound (I).

2. Process according to Claim 1 for preparing 4-(10,11-dihydro-3-fluoro-5H-dibenzo[a,d]cyclohepten-5-ylidene)-1-{2-[N-methyl-N-(2-pyrimidinyl)amino]ethyl}-piperidine.